(19) **Europäisches Patentamt / European Patent Office / Office européen des brevets**

(11) **EP 4 674 428 A1**

(12) **EUROPEAN PATENT APPLICATION**
published in accordance with Art. 153(4) EPC

(43) Date of publication:
07.01.2026 Bulletin 2026/02

(21) Application number: 24807141.7

(22) Date of filing: 10.05.2024

(51) International Patent Classification (IPC):
*A61K 38/40* (2006.01)    *A23L 33/19* (2016.01)
*A61P 7/06* (2006.01)    *A61P 13/12* (2006.01)
*A61P 21/00* (2006.01)

(52) Cooperative Patent Classification (CPC):
A23L 33/19; A61K 38/40; A61P 7/06; A61P 13/12;
A61P 21/00

(86) International application number:
PCT/JP2024/017376

(87) International publication number:
WO 2024/237189 (21.11.2024 Gazette 2024/47)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 12.05.2023 JP 2023079314

(71) Applicant: Tohoku University
Sendai-shi, Miyagi 980-8577 (JP)

(72) Inventors:
• SATO Emiko
  Sendai-shi, Miyagi 980-8577 (JP)
• IWAMOTO Yukina
  Sendai-shi, Miyagi 980-8577 (JP)

(74) Representative: Heyerhoff Geiger GmbH & Co. KG
Heiligenbreite 52
88662 Überlingen (DE)

Remarks:
The complete document including Reference Table(s) and the Sequence Listing(s) can be downloaded from the EPO website

(54) **COMPOSITION FOR USE IN TREATMENT AND/OR PREVENTION OF CHRONIC KIDNEY DISEASE COMPLICATIONS, KIDNEY DISORDER PROGRESSION INHIBITOR, SARCOPENIA PROGRESSION INHIBITOR, AND ANEMIA PROGRESSION INHIBITOR**

(57)    A problem is to provide a composition for use in treatment and/or prevention of a complication of chronic kidney disease, an inhibitor of progression of renal damage, an inhibitor of progression of sarcopenia and an inhibitor of progression of anemia.

The problem can be solved with a composition for use in treatment and/or prevention of a complication of chronic kidney disease containing lactoferrin as an active ingredient.

[FIG. 2]

KIDNEY WEIGHT/BODY WEIGHT

Mean ± SEM, Tukey-Kramer test, *p<0.05

EP 4 674 428 A1

## Description

Technical Field

The disclosure in the present application relates to a composition for use in treatment and/or prevention of a complication of chronic kidney disease, an inhibitor of progression of renal damage, an inhibitor of progression of sarcopenia and an inhibitor of progression of anemia.

Background Art

The kidneys are organs which excrete waste products from the blood, balance the water content and the contents thereof in the body, adjust blood acids and alkalis, secrete hematopoietic hormones, adjust the blood pressure and activate vitamin D, which involves in calcium metabolism. As a pathological condition of the kidneys, renal failure is known. Renal failure means a state of having renal dysfunction, mainly a decrease in the glomerular filtration rate (GFR). Renal failure is roughly classified into acute renal failure (ARF) and chronic renal failure (CRF) depending on the course of occurrence of the renal failure state. Chronic kidney disease (CKD) is a concept to view renal dysfunction as a disease at an earlier stage before becoming CRF, and acute kidney injury (AKI) is a concept including renal damage at an earlier stage for improving the prognosis of ARF.

Acute kidney injury is a pathological condition having azotemia, water/electrolyte abnormality or acid-base imbalance due to rapid renal impairment in several hours to several weeks and resulting breakdown of the mechanism of body fluid homeostasis. Acute kidney injury is defined as a condition in which the kidneys are changed functionally or structurally due to disorder caused by various factors and in which renal dysfunction has thus occurred rapidly (within 48 hours).

On the other hand, chronic kidney disease is defined as deterioration of the renal function indicated with the glomerular filtration rate (GFR) or as chronically lasting observation that suggests disorder of the kidneys.

Acute kidney injury is also known to develop into chronic kidney disease. Known pathways of the development of acute kidney injury into chronic kidney disease are (1) development of end-stage renal disease (ESRD) after the onset of acute kidney injury and transition to maintenance dialysis, (2) transition to chronic kidney disease due to incomplete recovery of the renal function, (3) transition to chronic kidney disease after temporal recovery of the renal function to the previous value from the onset of acute kidney injury, and the like.

When the renal function deteriorates, the QOL decreases because dialysis or kidney transplantation is required or because the risk of causing another disease becomes high. Accordingly, development of a drug which treats renal failure or prevents progression thereof is desired.

NPL 1 discloses that the renal function was recovered by lactoferrin and that renal fibrogenesis was inhibited by lactoferrin in a mouse model of transition from acute kidney injury to chronic kidney disease.

Citation List

Non Patent Literature

NPL 1: Yung-Ho Hsu et al., "Lactoferrin Contributes a Renoprotective Effect in Acute Kidney Injury and Early Renal Fibrosis", Pharmaceutics 2020, 12, 434

Summary of Invention

Technical Problem

Because the state with deteriorated renal function continues in chronic kidney disease, complications are frequently developed. However, the mechanisms of development are different between acute kidney injury and chronic kidney disease, and thus the model mice developing acute kidney injury described in NPL 1 have a problem because compositions for use in treatment and/or prevention of a complication of chronic kidney disease cannot be developed.

The disclosure in the present application has been made to solve the problem. As a result of extensive research, the present inventors have newly found that lactoferrin can be used for treating and/or preventing a complication of chronic kidney disease using adenine-induced renal failure model mice.

That is, an object of the disclosure of the present application is to provide a composition for use in treatment and/or prevention of a complication of chronic kidney disease, an inhibitor of progression of renal damage, an inhibitor of progression of sarcopenia and an inhibitor of progression of anemia.

Solution to Problem

(1) A composition for use in treatment and/or prevention of a complication of chronic kidney disease containing lactoferrin as an active ingredient.
(2) The composition according to (1) above
in which the treatment and/or the prevention of the complication of chronic kidney disease is inhibition of progression of sarcopenia and/or anemia through inhibition of progression of renal damage in chronic kidney disease.
(3) The composition according to (2) above
in which the inhibition of progression of renal damage is inhibition of progression of at least one selected from the group consisting of renal atrophy, a tubulointerstitial disease and renal impairment.
(4) The composition according to (2) above
in which the inhibition of progression of renal damage is inhibition of renal inflammation and/or fibrogenesis.
(5) The composition according to (2) above
in which the inhibition of progression of sarcopenia is inhibition of reduction in a muscle bundle section and/or a decrease in uremic toxin accumulation in a muscle.
(6) An inhibitor of progression of renal damage in chronic kidney disease containing lactoferrin as an active ingredient.
(7) An inhibitor of progression of sarcopenia in chronic kidney disease containing lactoferrin as an active ingredient.
(8) An inhibitor of progression of anemia in chronic kidney disease containing lactoferrin as an active ingredient.
(9) The composition according to any one of (1) to (5) above which is used as a component of a food composition or a component of a pharmaceutical composition.
(10) The inhibitor of progression according to any one of (6) to (8) above which is used as a component of a food composition or a component of a pharmaceutical composition.

Advantageous Effects of Invention

With the composition for use in treatment and/or prevention of a complication of chronic kidney disease, the inhibitor of progression of renal damage, the inhibitor of progression of sarcopenia and the inhibitor of progression of anemia disclosed in the present application, progression of renal damage, sarcopenia and anemia, which are complications of chronic kidney disease, can be prevented, or the progression can be inhibited.

Brief Description of Drawings

[FIG. 1] FIG. 1A is a graph showing the changes in the drinking amounts of the Adenine administration group, the lactoferrin administration group (Adenine + Lf administration group) and the Control group in experiment using mice without renal failure. FIG. 1B is a graph showing the changes in the body weights of the groups.
[FIG. 2] FIG. 2 shows pictures of the kidneys of the groups extracted during the dissection and a graph of the kidney weight/body weight.
[FIG. 3] FIG. 3 is a graph showing the changes in the blood urea nitrogen (BUN) of the groups.
[FIG. 4] FIG. 4 is a graph showing the measurement results of the blood creatinine concentrations of the groups.
[FIG. 5] FIG. 5 shows graphs showing the blood concentrations of indoxyl sulfate, hippuric acid and p-cresyl sulfate of the groups.
[FIG. 6] FIG. 6 shows pictures of the Masson trichrome-stained kidneys of the groups and a graph showing the remaining renal tubule rates.
[FIG. 7] FIG. 7 shows pictures of the H&E-stained kidneys of the groups and a graph showing the crystal rates.
[FIG. 8] FIG. 8 is a graph showing the F4/80 positive rates of the groups.
[FIG. 9] FIG. 9 shows graphs showing the changes in hemoglobin and hematocrit in the blood collected from the veins of the cheeks of the groups.
[FIG. 10] FIG. 10 shows pictures of the H&E-stained left femoris muscles of the groups and graphs showing the muscle cross sectional areas.
[FIG. 11] FIG. 11 shows graphs showing the concentrations of indoxyl sulfate and hippuric acid in the muscles of the groups.
[FIG. 12] FIG. 12 is a graph showing the expression of p47phox gene, which is an indicator of oxidative stress, in the kidneys of the groups.
[FIG. 13] FIG. 13 shows graphs showing kidney weight/body weight and heart weight/body weight of those extracted during the dissection of the group without lactoferrin administration (Adenine), the lactoferrin administration group (Adenine + Lf) and the control group (Control) in experiment using mice with renal failure.

[FIG. 14] FIG. 14 is a graph showing the blood concentrations of indoxyl sulfate of the groups.

[FIG. 15] FIG. 15 is a graph showing the results of enrichment analysis by MetaboAnalyst 5.0.

[FIG. 16] FIG. 16 shows graphs showing the comparison of the valine, leucine and isoleucine amounts in the quadriceps femoris muscle among the three groups.

[FIG. 17] FIG. 17 shows the results of western blotting of p70S6K and phosphorylated p70S6K in the quadriceps femoris muscle and a graph showing the comparison of the phosphorylation rates among the three groups, where the phosphorylated p70S6K amounts relative to the expression levels of p70S6K were quantified by quantifying the band intensities, and the quantified values were compared based on that of the Control regarded as 1.

[FIG. 18] FIG. 18 shows the results of western blotting of LC3 in the quadriceps femoris muscle and a graph showing the comparison of the expression levels among the three groups, where the band intensities of LC3-II were quantified and corrected with β-actin, and the quantified values were compared based on that of the Control regarded as 1.

[FIG. 19] FIG. 19 shows the results of western blotting of cathepsin B in the quadriceps femoris muscle and a graph showing the comparison of the expression levels among the three groups, where the band intensities of cathepsin B were quantified and corrected with β-actin, and the quantified values were compared based on that of the Control regarded as 1.

[FIG. 20] FIG. 20 shows the results of western blotting of AMPKα and phosphorylated AMPKα in the quadriceps femoris muscle and a graph showing the comparison of the phosphorylation rates among the three groups, where the phosphorylated AMPKα amounts relative to the expression levels of AMPKα were quantified by quantifying the band intensities, and the quantified values were compared based on that of the Control regarded as 1.

[FIG. 21] FIG. 21 explains the schedule of the experiment on therapeutic effects of Lf on chronic kidney disease using C57BL/6JJcl males.

[FIG. 22] FIG. 22 shows pathological pictures of typical Elastica·Masson-stained kidneys and a graph showing the comparison of the quantified remaining renal tubule rates among the three groups.

[FIG. 23] FIG. 23 shows graphs showing the comparison of the expression levels of fibrogenesis-related genes in the kidneys among the three groups.

[FIG. 24] FIG. 24 shows pictures of typical cross sections of H&E-stained quadriceps femoris muscles, a graph showing the comparison of the quantified muscle cross sectional areas among the three groups and the distributions of the muscle cross sectional areas.

[FIG. 25] FIG. 25 shows a graph showing the comparison of the indoxyl sulfate concentrations in the quadriceps femoris muscle among the three groups.

Description of Embodiments

The composition for use in treatment and/or prevention of a complication of chronic kidney disease, the inhibitor of progression of renal damage, the inhibitor of progression of sarcopenia and the inhibitor of progression of anemia disclosed in the present application are explained in detail below. In the present specification, numerical values, numerical ranges and qualitative expressions (for example, expressions such as "identical" and "same") indicate the numerical values, the numerical ranges and the properties including errors that are generally accepted in the technical field.

(Embodiment of Composition for Use in Treatment and/or Prevention of Complication of Chronic Kidney Disease, Inhibitor of Progression of Renal Damage, Inhibitor of Progression of Sarcopenia and Inhibitor of Progression of Anemia)

The composition for use in treatment and/or prevention of a complication of chronic kidney disease, the inhibitor of progression of renal damage, the inhibitor of progression of sarcopenia and the inhibitor of progression of anemia (sometimes referred to as "composition/inhibitor" below for the characteristics common among the composition and the progression inhibitors) contain lactoferrin as an active ingredient. In the present specification, the treatment means relief of a symptom of a developed complication of chronic kidney disease or inhibition of progression thereof. Moreover, the prevention means prevention or delay of the onset of a complication of chronic kidney disease or inhibition of progression of the onset thereof.

Lactoferrin is an iron-binding glycoprotein contained in milk, tears, saliva, blood and the like of a mammal, for example, sheep, goat, pig, mouse, water buffalo, camel, yak, horse, donkey, llama, cow or human. As the lactoferrin contained in the composition/inhibitor according to the embodiment, lactoferrin derived from any of the mammals above or a mammal which is not cited as an example can be used. In view of the content and the easiness of acquisition, rather than technical view, for example, lactoferrin derived from milk of a cow, a human or the like is a preferable example. Examples of the milk include colostrum, transitional milk, normal milk and late lactation milk.

The lactoferrin may be lactoferrin separated from skim milk, whey and the like, which are treated milk products, by a normal method (for example, ion chromatography or the like), recombinant lactoferrin produced by a microorganism, animal cells, a transgenic animal or the like through genetic engineering, synthetic lactoferrin or a mixture thereof. Moreover, the lactoferrin may be non-glycosylated or glycosylated lactoferrin. As such lactoferrin, commercial lactoferrin which is manufactured in an industrial scale can be used.

The metal content of the lactoferrin is not particularly limited, and any one kind or a mixture of two or more kinds selected from the group consisting of apo-lactoferrin, which is obtained by removing iron from lactoferrin using hydrochloric acid, citric acid or the like, metal-saturated lactoferrin having a saturation degree of 100% or more, which is obtained by chelating apo-lactoferrin with metals such as iron, copper, zinc and manganese, and partially metal-saturated lactoferrin, to which metals are bonded with saturation degrees of less than 100%, can be used.

Although it is not limited, an example of the preparation (separation or purification of lactoferrin from a raw material such as milk) method of lactoferrin is as follows. A raw milk material is caused to pass through a cation-exchange column, and the liquid which has passed through the column is recovered and appropriately caused to pass repeatedly through the column. Deionized water is caused to pass through the column, and sodium chloride solution is caused to pass through the column to obtain an eluate of basic protein adsorbed to the cation-exchange column. This is recovered from the eluate by ammonium sulfate precipitation and appropriately washed. The recovered precipitate is dissolved in deionized water, and the solution is filtered with an ultrafiltration membrane. Furthermore, through desalination treatment and freeze-drying, powdery lactoferrin is obtained. Of course, commercial prepared lactoferrin may also be used.

Lactoferrin sometimes has genetic mutation, such as replacement, deletion, insertion or addition of one or more bases and inversion, at one or more locations, depending on the difference in species, genus, individual or the like. Mutation is sometimes included also in the amino acids of the protein encoded by the gene having such mutation. The lactoferrin contained in the composition/inhibitor according to the embodiment may include such mutation within the range in which the effects of the composition/inhibitor are exerted. Moreover, within the range in which the effects of the composition/inhibitor are exerted, the lactoferrin may be treated lactoferrin which has undergone heat treatment, acid treatment or alkali treatment, or treated lactoferrin may be contained as a part thereof.

Examples of the dosage form of the composition/inhibitor according to the embodiment include tablets, pills, powder, lozenges, a sachet, a cachet, an elixir, a suspension, an emulsion, a solution, a syrup, an aerosol (as a solid or in a liquid medium), an ointment, soft and hard gelatin capsules, a suppository, eye drops, nasal drops, a plaster, a sterilized solution for injection, and sterilized encapsulated powder.

Moreover, the composition/inhibitor according to the embodiment may contain a carrier, an excipient or a diluent. Examples thereof include lactose, dextrose, sucrose, sorbitol, mannitol, starch, gum acacia, calcium phosphate, alginate, tragacanth, gelatin, calcium silicate, microcrystalline cellulose, polyvinylpyrrolidone, cellulose, water, phosphate-buffered saline (PBS), syrup, methylcellulose, oxy methyl and propyl benzoate, talc, magnesium stearate and mineral oil. Furthermore, a lubricant, a moisturizer, an emulsifier, a suspending agent, a preservative, a sweetener or an aromatizing agent may be added. Moreover, when the composition/inhibitor according to the embodiment is administered by injection, a known medicine, such as water-soluble hyaluronic acid, a steroid, an anesthetic and an anti-inflammatory agent, may be combined depending on the injection site.

The dose of the composition/inhibitor according to the embodiment may be appropriately determined depending on the purpose. Here, in the homepage of the Clinical Lactoferrin Society (http://www.clinical-lactoferrin.com/), it is written that lactoferrin is administered at 100 mg to 1,200 mg/day as a reference, although it depends on the disease. Although it is not limited, referring to the description, the lactoferrin amount contained in the composition/inhibitor may be determined in such a manner that around 100 mg to 1,200 mg can be taken per day in the case of a human.

The composition/inhibitor according to the embodiment can be used as a component of a pharmaceutical composition or a component of a food composition.

First, a case in which the composition/inhibitor according to the embodiment is used as a component of a food composition is explained. As long as the composition/inhibitor according to the embodiment is contained as a component of a food composition, the other components of the food composition are not particularly restricted. The food composition containing the composition/inhibitor according to the embodiment can be provided as a functional food such as foods for specified health uses having efficacy such as prevention of a complication of chronic kidney disease, inhibition of progression of renal damage in chronic kidney disease, inhibition of progression of sarcopenia in chronic kidney disease and inhibition of progression of anemia in chronic kidney disease. The intake for obtaining such efficacy may be appropriately determined considering that the food, for example, the functional food, is taken daily, continuously or intermittently for a long time.

Although it is not limited, similar to the composition/inhibitor, the lactoferrin amount contained in the food composition may be determined in such a manner that around 100 mg to 1,200 mg can be taken per day in the case of a human. The intake period of the food composition is not particularly restricted, but in view of prevention or inhibition of progression, the food composition is preferably taken for a long time. Although it is not limited, examples thereof include one week or longer, two weeks or longer, three weeks or longer, one month or longer, three months or longer, six months or longer, and one year or longer.

The food composition (including drinking water) can be in any form of a solid, a gel and a liquid. Examples thereof include a fermented milk product such as lactic acid bacteria beverages, processed foods and drinks, dry powder, tablets, capsules, and granules, furthermore, the food composition can be a drink, yogurt, a liquid food, a jelly, a candy, a retort-pouched food, a tablet candy, a cookie, a sponge cake, bread, a biscuit, chocolate or the like. Moreover, an additive such as a saccharide, protein, lipid, a vitamin, a mineral, a flavor, a carbohydrate, a sweetener, an aroma, a dye, a texture-improving agent and a mixture thereof may be added to the food composition to improve the nutritional balance, the flavor and the like.

Next, a case in which the composition/inhibitor according to the embodiment is used as a component of a pharmaceutical composition is explained. As long as the composition/inhibitor according to the embodiment is contained as a component of a pharmaceutical composition, the other components of the pharmaceutical composition are not particularly restricted. The pharmaceutical composition containing the composition/inhibitor according to the embodiment can be provided as a pharmaceutical composition having efficacy such as prevention of a complication of chronic kidney disease, inhibition of progression of renal damage in chronic kidney disease, inhibition of progression of sarcopenia in chronic kidney disease and inhibition of progression of anemia in chronic kidney disease or as a pharmaceutical composition to which the efficacy has been added.

The pharmaceutical composition can be administered locally or administered systemically. The administration form is not particularly limited and may be either of oral administration and parenteral administration (for example, eye drops, nasal administration or the like). Moreover, the pharmaceutical composition can contain a pharmacologically acceptable carrier depending on the administration form in addition to lactoferrin as the active ingredient. Examples of the carrier include an excipient, a disintegrator or a disintegrating aid, a binder, a lubricant, a coating agent, a dye, a diluent, a base, a solubilizer or a solubilizing aid, an isotonic agent, a pH-adjusting agent, a stabilizer, a sprayer, and a tackifier.

The dose of the pharmaceutical composition varies with the body weight, the age and the like of the patient. The dose is not particularly limited. The dose may be appropriately determined by a doctor. Moreover, similar to the food composition, as a rough reference, the lactoferrin amount contained in the pharmaceutical composition may be determined in such a manner that around 100 mg to 1,200 mg can be administered per day in the case of a human.

In this regard, the animal which develops chronic kidney disease is not limited to human, and a non-human animal such as dog, cat, horse and birds also develops chronic kidney disease. The composition/inhibitor disclosed in the present application can also be used for a non-human animal. The dose may be appropriately adjusted depending on the body weight or the like.

Examples of the complication of chronic kidney disease include disorder of urinary concentrating capacity, azotemia, water/electrolyte abnormality (excessive body fluid or hyperkalemia), metabolic acidosis and secondary hyperparathyroidism as well as renal anemia caused by renal damage, sarcopenia (muscle atrophy), bone disorder and cognitive impairment, which are caused by uremic toxins that accumulate in the body due to renal impairment, and the like. The composition/inhibitor according to the embodiment treats such a complication or prevents or inhibits progression thereof.

A more specific example of the inhibition of progression of renal damage is inhibition of progression of renal atrophy, a tubulointerstitial disease or renal impairment. Another example is inhibition of progression of renal inflammation and/or fibrogenesis. In the present specification, renal atrophy means the state in which the kidneys shrink to around a half of the normal state and fall into dysfunction. A tubulointerstitial disease means a disorder in the interstitium, which is a tissue among renal tubules (a tissue that plays a role of re-absorbing and returning components necessary for the body, such as water and electrolyte, in the urine excreted from glomeruli to the blood and excreting unnecessary components as urine). Renal impairment means that the glomerular filtration rate becomes less than 60 ml/min/1.73 $m^2$. Renal inflammation means inflammation induced when immunological mechanism due to immune response to infection or various types of allergic reaction to drugs or the like involves and causes inflammatory cells such as macrophages and lymphocytes to infiltrate to the interstitium and when the activated macrophages then produce inflammatory cytokines. Renal fibrogenesis means that the interstitial area becomes fibrous.

Sarcopenia (muscle atrophy) means a disease characterized by a decrease in the muscle amount and a decrease in the muscle strength. More specifically, sarcopenia means reduction in a muscle cross section and/or uremic toxin accumulation in a muscle.

Anemia means a decrease in hemoglobin and the hematocrit level, palpitation, shortness of breath, dizziness and the like which develop because a hematopoietic hormone (erythropoietin) produced in the kidneys decreases.

Next, the differences between the adenine-induced renal failure model mice used in the Example of the present application and the model mice described in NPL 1 are explained.

The adenine-induced renal failure model mice used in the Example of the present application are model mice with progression of renal damage caused by tubulointerstitial nephritis, in which monocytes/macrophages produce cytokines and cause fibrogenesis due to lasting inflammation caused by gradual crystallization of an adenine metabolite, 2,8-dihydroxyadenine (2,8-DHA), in renal tubules through administration of adenine. The adenine-induced renal failure model mice directly develop a pathological condition of chronic kidney disease (CKD) without undergoing the phase of acute kidney injury (AKI). Accordingly, the model is not suitable for inducing acute kidney injury.

On the other hand, NPL 1 uses model mice which shift to chronic kidney disease from acute kidney injury through induction with folic acid. In the model mice of NPL 1, acute necrosis of renal tubules (loss of the brush border of proximal tubules, vacuolization, detachment or coagulation necrosis of renal tubular epithelial cells and the like) is caused through rapid crystal formation after administration of a high dose of folic acid, and then renal tubular atrophy and fibrogenesis of interstitium are caused without recovery from the renal tubular necrosis, resulting in transition to chronic kidney disease.

As described above, the mechanisms of the onset of renal failure are totally different between the mice used in the present application and the mice described in NPL 1. As shown in the Example described below, it has been indicated that lactoferrin has totally different mechanisms of action in the mice used in the present application and in the mice described in NPL 1.

Moreover, in addition to the model mice described in NPL 1, spontaneous type 2 diabetes model mice which develop renal damage (mainly glomerular disorder) caused by hyperglycemia accompanying diabetes are also known. The spontaneous type 2 diabetes model mice are mice in which proteinuria and renal dysfunction are caused by the damage of the glomerulus (growth of mesangial cells, infiltration of inflammatory cells into glomerulus, disorder of glomerular capillary walls and the like). The spontaneous type 2 diabetes model mice also have a different mechanism of the onset of the pathological condition from that of the model mice used in the present application.

Here, the purposes and the methods of treatment are different between acute renal failure and chronic renal failure. While the renal dysfunction of acute renal failure is expected to be reversible, chronic renal failure is irreversible. Accordingly, treatment aiming at recovery of the renal function is conducted for acute renal failure, and the purpose of the treatment of chronic renal failure is inhibition of progression of renal dysfunction. More specifically, in acute renal failure, drug therapy for removing the cause and symptomatic treatment up to the recovery of the renal function are conducted. On the other hand, in chronic renal failure, drug therapy for controlling the risk factors for hypertension, diabetes and the like and symptomatic treatment for maintaining the QOL are conducted. For example, a drug for improving abnormality accompanying long-term progress (anemia, bone metabolism disorder or the like) is required only for chronic renal failure.

As explained above, between acute renal failure and chronic renal failure, the mechanisms of the onset of the pathological condition are different, and the purposes and the methods of treatment are also different. Accordingly, for screening of drugs for treating or preventing renal failure or inhibiting progression thereof, it is required to select suitable model mice according to the purpose, considering the mechanism of the onset of renal failure in addition to the target, namely acute renal failure or chronic renal failure. In the disclosure of the present application, using adenine-induced renal failure model mice in view of treatment and/or prevention of a complication of chronic kidney disease, it has been newly found that lactoferrin treats and/or prevents a complication of chronic kidney disease.

The embodiments disclosed in the present application are explained specifically below referring to an Example, but the Example is merely for explaining the embodiments. It is not intended that the Example limits or restricts the scope of the invention disclosed in the present application.

Examples

[Experimental Animals and Experiment Procedures: Examination of Prevention Effects on Complications of Chronic Kidney Disease]

<Experimental Animals>

· Mice: Seven-week-old C57BL/6JJcl male mice purchased from CLEA Japan, Inc. and habituated for one week were used.

<Experiment Procedures>

The eight-week-old mice were randomly divided into three groups of

· a renal failure group (Adenine administration group),

· a lactoferrin administration group (Adenine + Lf administration group) and
· a control group (Control group)

(n=6 in each group).

MF (Oriental Yeast Co., Ltd.) containing 0.2% adenine (A8626, Merck, New Jersey, USA) (adenine food) was given to the renal failure group for six weeks. The 0.2% adenine-containing MF food was prepared by Oriental Yeast Co., Ltd. Moreover, tap water was given by free drinking.

A control MF food containing no adenine was given to the control group for six weeks. Moreover, tap water was given by free drinking.

MF (Oriental Yeast Co., Ltd.) containing 0.2% adenine (A8626, Merck, New Jersey, USA) (adenine food) was given to the lactoferrin administration group for six weeks as in the renal failure group. To the lactoferrin administration group, an aqueous 2% lactoferrin solution was given by free drinking instead of tap water. The aqueous lactoferrin solution used was prepared at 2% by completely dissolving 10 g of Lf made in Australia (NRL Pharma, Inc.) in 500 mL of tap water.

After six weeks had passed, the mice were euthanized under isoflurane anesthesia, and the blood was collected. After whole body perfusion with 0.9% saline, kidney and muscle samples were collected.

[Inhibition of Adenine Administration-Caused Body Weight Decrease by Lactoferrin]

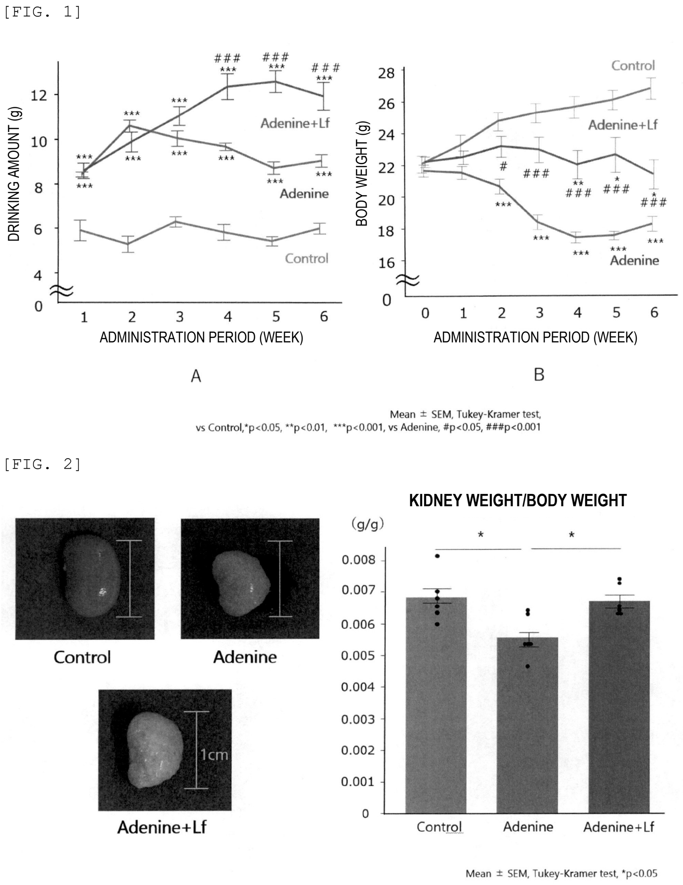

FIG. 1 shows graphs showing the changes in the drinking amounts and the body weights of the groups. As shown in FIG. 1A, the drinking amount was increased by the adenine administration. As shown in FIG. 1B, while the body weight decreased in the group to which adenine was administered compared to that of the control group, the decrease in the body weight was inhibited by the administration of lactoferrin in the lactoferrin administration group.

[Inhibition of Adenine Administration-Caused Renal Atrophy by Lactoferrin]

In FIG. 2, pictures of the kidneys extracted during the dissection and a graph of the kidney weight/body weight are shown. As it is obvious from FIG. 2, it was found that lactoferrin inhibits renal atrophy caused by adenine administration.

[Inhibition of Adenine Administration-Caused Deterioration of Renal Function and Inhibition of Increase in Blood Uremic Toxins by Lactoferrin]

<Quantification of Blood Urea Nitrogen (BUN)>

For measuring the urea nitrogen, the mouse plasma was diluted 20 folds or 200 folds with ultrapure water. The measurement was conducted as follows using BUN colorimetric kit (Arbor assays, nn Arbor, NC, USA) according to the instruction manual.

A 96-well transparent plate (1-1601-02; AS ONE Corporation) was used, and the procedure was performed according to the manual. The processed samples were incubated at room temperature for 30 minutes, the absorbance at 450 nm was measured using Spectra Max 190 (Molecular Devices, San Jose, CA, USA). A calibration curve was created by 4-parameter logistic regression analysis, and the concentration of urea nitrogen was calculated from the measured absorbance.

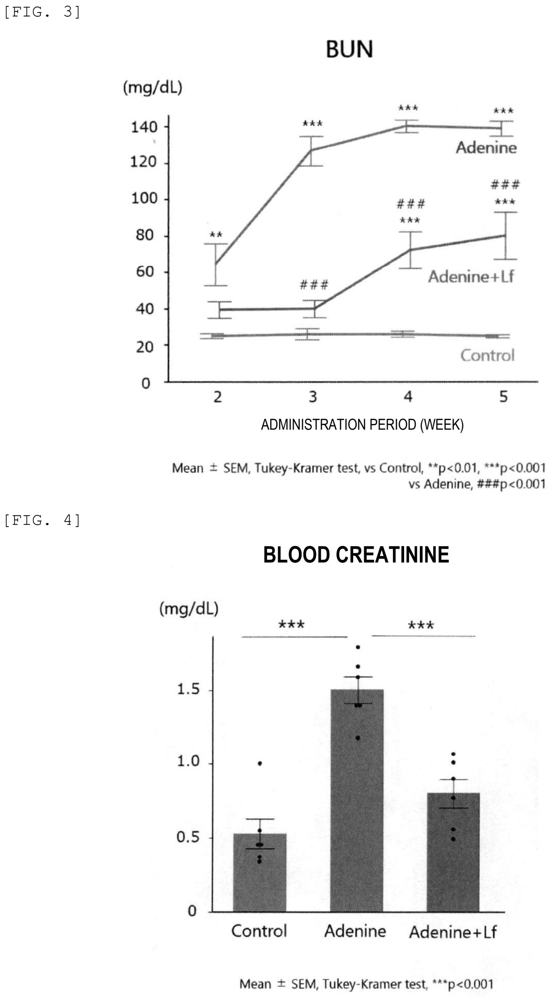

The results are shown in FIG. 3. As it is obvious from FIG. 3, the increase of blood urea nitrogen level was inhibited by the administration of lactoferrin in the lactoferrin administration group.

<Measurement of Plasma Creatinine and Uremic Toxins>

To 50 $\mu$L of mouse plasma, 150 $\mu$L of 0.1% formic acid methanol (containing 1.25 $\mu$g/mL 3-indoxyl sulfate-$d_4$ and 1 $\mu$g/mL creatinine-$d_3$) was added and mixed by vortexing. Then, ultrasonic treatment was conducted for five minutes, and centrifugation was conducted at 16,400$\times$ g for 20 minutes at 4°C. The obtained supernatant was filtered with a 0.2-$\mu$m filter (YMC Duo-Filter, YMC, Kyoto, Japan).

The IS (indoxyl acid) concentration was measured by liquid chromatography tandem mass spectrometry (LC-MS/MS). The LC part was NANOSPACE SI-2 (Shiseido Company, Limited, Tokyo, Japan), and the MS part was TSQ Quantum Ultra (Thermo Fisher Scientific, San Jose, CA, USA). The measurement was conducted with the negative mode, and 150$\times$2.0 mm YMC-Pack Pro C18 (#AS12S03-1502WT; YMC, Kyoto, Japan) was used as the column. The mobile phases were a solvent A) 10 mM ammonium acetate and a solvent B) acetonitrile. The flow rate was 0.3 ml/min, and the measurement was conducted with the following gradient.

0-3 min: 0-50% solvent B;
3.1-4 min: 50-80% solvent B;
4.1-5 min: 80-100% solvent B;
5.1-7 min: 100% solvent B;
7.1-10 min: 0% solvent B.

IS and IS-d$_4$ were measured with SRM (selected reaction monitoring). IS was detected with a parent ion at m/z 212, a product ion at m/z 80, a collision energy of 21 V and T-Lens of 112 V, and IS-d$_4$ was detected with a parent ion at m/z 216, a product ion at m/z 80 and a collision energy of 30 V.

Hippuric acid was detected with a parent ion at m/z 178, a product ion at m/z 134, a collision energy of 13 V and T-Lens of 71 V, and hippuric acid-ds was detected with a parent ion at m/z 183, a product ion at m/z 139, a collision energy of 27 V and T-Lens of 71 V.

p-Cresyl sulfate was detected with a parent ion at m/z 187, a product ion at m/z 107, a collision energy of 23 V and T-Lens of 44 V, and p-cresyl sulfate-d$_4$ was detected with a parent ion at m/z 191, a product ion at m/z 110, a collision energy of 30 V and T-Lens of 44 V. The spray voltage, the vaporizer temperature and the capillary temperature were 2,500 V, 450°C and 220°C, respectively.

The blood creatinine was measured with LC-MS/MS in which NANO SPACE SI-2 (Shiseido) and TSQ Quantum Ultra (Thermo Fisher Science) were connected. As the mobile phases, a solvent A) 0.1% formic acid and a solvent B) acetonitrile were used, and the elution was conducted with the following concentration gradient.

0-1 min; B 2%-5%,
1-3 min; 5%-20%,
5.1-7 min; 50%-100%,
7.1-10 min; 100%.

As the column, YMC-Pack Pro C18 150×2.0 mm ID (YMC) was used. The flow rate was 0.2 mL/min, and the sample injection amount was 3 μL. Regarding the SRM conditions, creatinine was detected with a parent ion at m/z 114, a product ion at m/z 86, a collision energy of 11 V and T-Lens of 100 V, and creatinine-d$_3$ was detected with a parent ion at m/z 117, a product ion at m/z 89, a collision energy of 12 V and T-Lens of 100 V. The measurement mode was the positive mode. The spray voltage, the vaporizer temperature and the capillary temperature were 3,000 V, 350°C and 350°C, respectively.

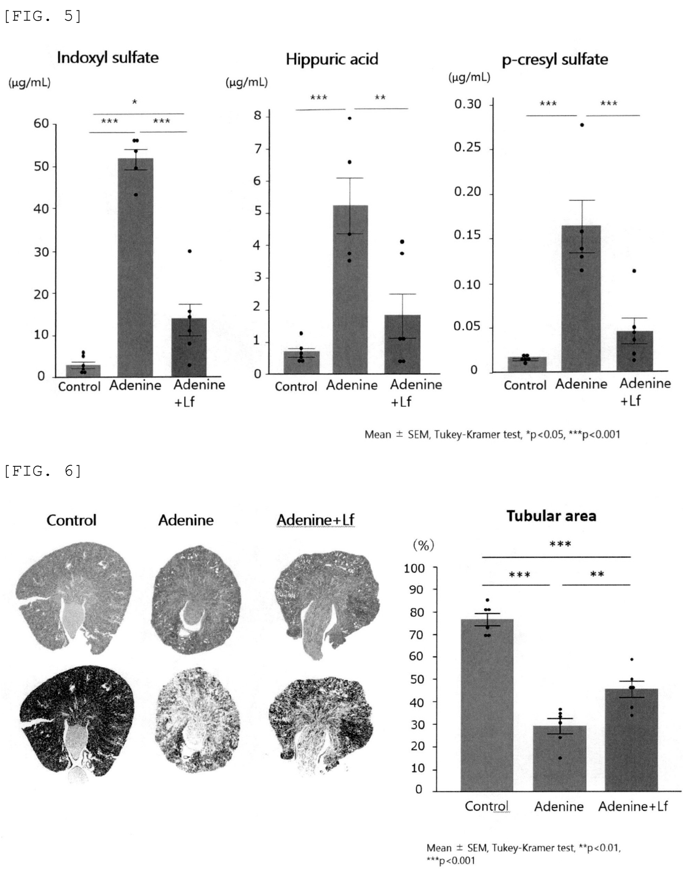

The measurement results of the blood creatinine concentrations are shown in FIG. 4. The blood concentrations of indoxyl sulfate, hippuric acid and p-cresyl sulfate are shown in FIG. 5. From the results shown in FIG. 3 and FIG. 4, it was found that lactoferrin inhibits deterioration of the renal function caused by adenine administration. From the results shown in FIG. 5, it was found that lactoferrin inhibits an increase in blood uremic toxins caused by renal failure.

[Renal Histologic Analysis]

Next, histologic analysis of the kidneys was conducted.

<Masson Trichrome Staining>

The kidneys extracted during the dissection were fixed with 4% paraformaldehyde (PFA) (168-23255; Wako Pure Chemical Industries, Ltd.) and sliced into 2 μm after paraffin embedding.

For Masson trichrome staining, Masson-Golder staining kit (100485; Sigma-Aldrich, Co., St. Louis, MO, USA) was used.

Paraffin was removed from each kidney slice on a slide with 80% xylene and 99.5% ethanol, and then the nuclei were stained with hematoxylin for five minutes. After washing with tap water for five minutes, the kidney slice was washed with an aqueous 1% acetic acid solution (01021-00; KANTO CHEMICAL CO., INC.) for 30 seconds and immersed in and reacted with Azophloxine solution for 10 minutes. The kidney slice was washed with 1% acetic acid for 30 seconds and immersed in tungstophosphoric acid orange G solution for one minute. Next, the kidney slice was washed with 1% acetic acid for 30 seconds, immersed in Light green SF solution for two minutes and then washed again with 1% acetic acid for 30 seconds. The kidney slice was dehydrated and cleared in three 80% xylene baths and four 99.5% ethanol baths and mounted using Entellan (registered trademark) New.

<Evaluation of Tubulointerstitial Disease>

Using each Masson trichrome-stained specimen, the remaining renal tubular area which was stained in red and the cortex area were quantified using Image J. The remaining renal tubule rate was calculated using the calculation equation below, and thus the interstitial disease of proximal tubules was evaluated. The results are shown in FIG. 6.

$$\text{Remaining renal tubule rate (\%)} = \frac{\text{Remaining renal tubular area}}{\text{Cortex area}} \times 100$$

<Evaluation of Crystal Area>

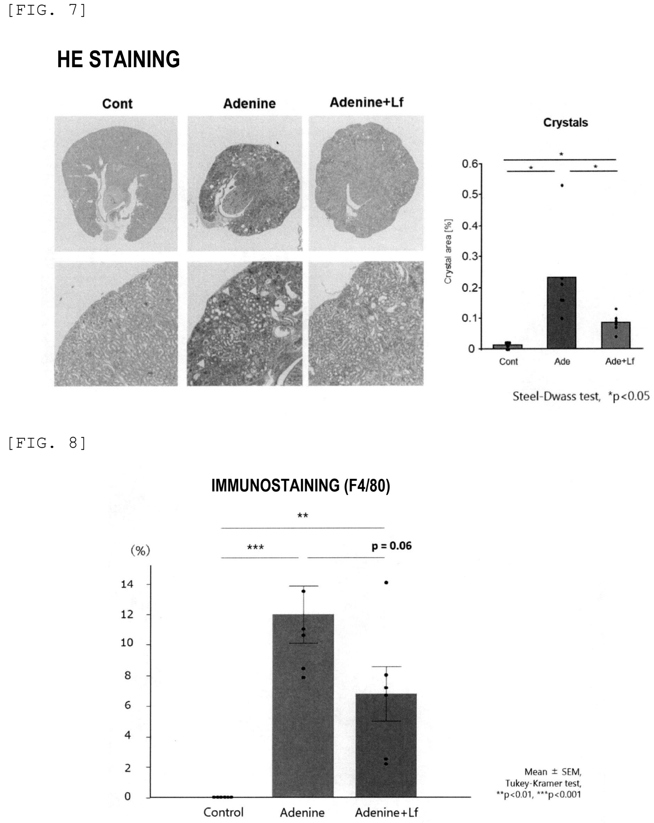

Using each hematoxylin eosin (HE)-stained specimen, the crystals of 2,8-dihydroxyadenine were quantified using Image J. The area in which crystals were formed was calculated using the calculation equation below, and thus the crystal production was evaluated. The results are shown in FIG. 7.

$$\text{Crystal rate } = \frac{\text{Crystal area}}{\text{Cortex area}} \times 100$$

From the results shown in FIG. 6, it was found that lactoferrin inhibits the tubulointerstitial disease caused by adenine administration. Moreover, from the results shown in FIG. 7, it was found that lactoferrin inhibits crystal formation in renal tubules caused by adenine administration.

<Immunostaining>

The kidneys extracted during the dissection were fixed with 4% paraformaldehyde (PFA) (168-23255; Wako Pure Chemical Industries, Ltd.) and sliced into 2 $\mu$m after paraffin embedding. Paraffin was removed from each kidney slice on a slide with 80% xylene and 99.5% ethanol, and after washing with PBS, the kidney slice was treated with Proteinase K (S3020, DAKO) for protease treatment for five minutes at normal temperature. The slide was washed with PBS and then immersed in 0.3% hydrogen peroxide methanol for 15 minutes for blocking the endogenous peroxidase activity. The slide was washed with PBS and then treated with Protein Block Serum-Free (X0909, DAKO) for 60 minutes at normal temperature. The slide was washed with PBS and then reacted with a primary antibody, Rat anti Mouse F4/80 (MCA497, SRT Bio-Rad), which was diluted 200 folds overnight at 4°C. The slide was washed with PBS containing 0.3% tween-20 and then reacted with a secondary antibody, simple stain mouse (MAX-PO (Rat)), for 60 minutes at room temperature. The slide was washed with PBS containing 0.3% tween-20 and then reacted with ImmPACT DAB substrate kit (sk-4105, Vector) for six minutes at room temperature for color development. The slide was washed with distilled water, and then the nuclei were stained with hematoxylin. After dehydration and clearing with ethanol and xylene, the slide was mounted.

[Evaluation of F4/80 Positive Rate (Inhibition of Adenine Administration-Caused Renal Inflammation by Lactoferrin)]

An image of each specimen which was subjected to F4/80 immunostaining was taken with Keyence BZ-X800, and the F4/80 positive area was quantified using Image J. The F4/80 positive rate was calculated using the calculation equation below, and thus the infiltration of macrophages was evaluated. The results are shown in FIG. 8.

$$\text{F4/80 positive rate (\%) } = \frac{\text{F4/80 positive area}}{\text{Cortex area}} \times 100$$

From the results shown in FIG. 8, it was found that lactoferrin has a tendency to inhibit renal inflammation caused by adenine administration.

[Inhibition of Adenine Administration-Caused Anemia by Lactoferrin]

<Hemocyte Calculation>

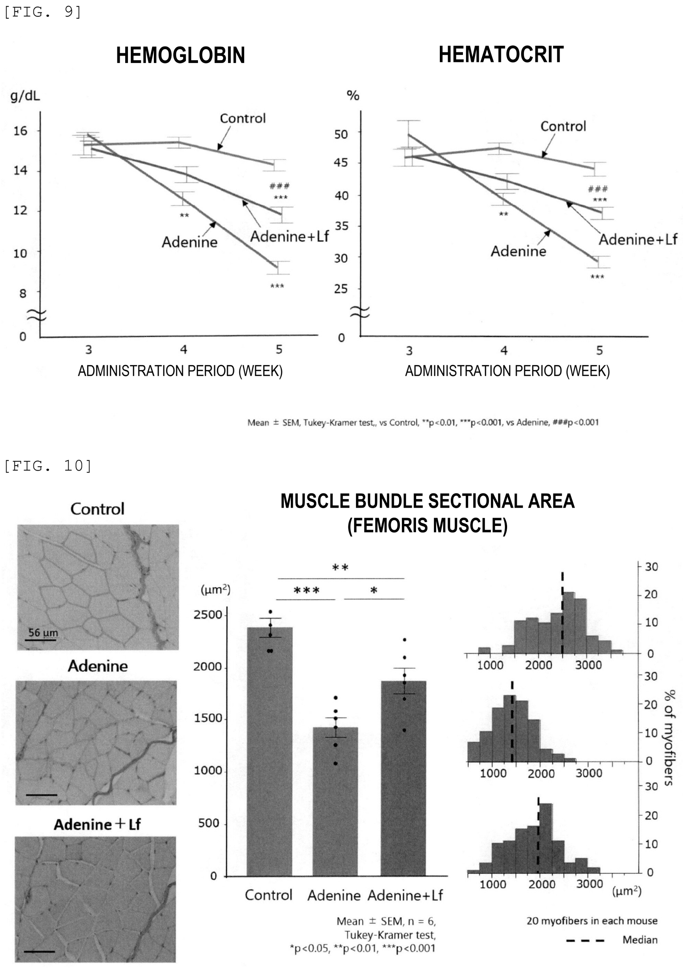

EDTA was used as a coagulant, and a blood sample was taken from a vein in the cheek three, four and five weeks after starting the experiment. For the measurement for calculating hemocytes, 20 $\mu$L of blood was used. For hemocyte measurement, HORIBA Microsemi LC-662 hemocyte count analyzer was used. The results are shown in FIG. 9. As it is obvious from FIG. 9, it was found that lactoferrin inhibits decreases in hemoglobin and hematocrit caused by adenine administration. From the above results, it was found that lactoferrin inhibits anemia caused by adenine administration.

[Inhibition of Adenine Administration-Caused Sarcopenia by Lactoferrin]

<Evaluation of Muscle Cross Section>

The left femoris muscle extracted during the dissection was fixed with 4% paraformaldehyde (PFA) (168-23255; Wako Pure Chemical Industries, Ltd.), sliced into 2 $\mu$m after paraffin embedding and stained with H&E. Images of the specimens were taken with Keyence BZ-X800 (KEYENCE CORPORATION., Osaka, Japan). Twenty muscle cross sections were randomly selected from each specimen and used for the analysis. For image analysis, Image J software was used.

The results are shown in FIG. 10. As it is obvious from FIG. 10, it was found that lactoferrin inhibits reduction in a muscle cross section caused by renal failure induced by adenine administration.

[Inhibition of Adenine Administration-Caused Sarcopenia by Lactoferrin]

Femoral muscle in an amount of 40 to 120 mg was weighed in a 2.0-mL tube (TM-625), and 0.1% formic acid methanol (containing 1.25 mg/mL 3-indoxyl sulfate-$d_4$ and 1 mg/mL creatinine-$d_3$) and three zirconia beads were added. The amount to be added was determined by the following calculation.

$$\texttt{Addition amount = tissue amount (g)} \times \texttt{1000} \times \texttt{4 (μL)}$$

Next, the tissue was homogenized with Micro Smach MS-100 (TOMY SEIKO, Tokyo, Japan) at 5,000 rpm for 30 seconds and subjected to ultrasonic treatment for 10 minutes at normal temperature. After centrifugation at 16,400× g for 20 minutes at 4°C, 100 μL of the supernatant was transferred to a 1.5-mL tube and vortexed after adding 100 μL of 10 mM ammonium acetate. After transferring 100 μL thereof to a 1.5-mL tube, the sample was dried by drying under reduced pressure for two hours and 30 minutes and dissolved again in 10 μL of 10 mM ammonium acetate. The sample was vortexed for one minute and centrifuged at 16,400× g for three minutes at 4°C, and 10 μL thereof was applied to 0.2 μm Filter (YMC Duo-Filter, YMC, Kyoto, Japan) and centrifuged at 7,000 rpm for three minutes at 4°C. To the filtered sample, 5 μL of 10 mM ammonium acetate was added, and using this as a sample, uremic toxins in the muscle were measured by LC-MS/MS.

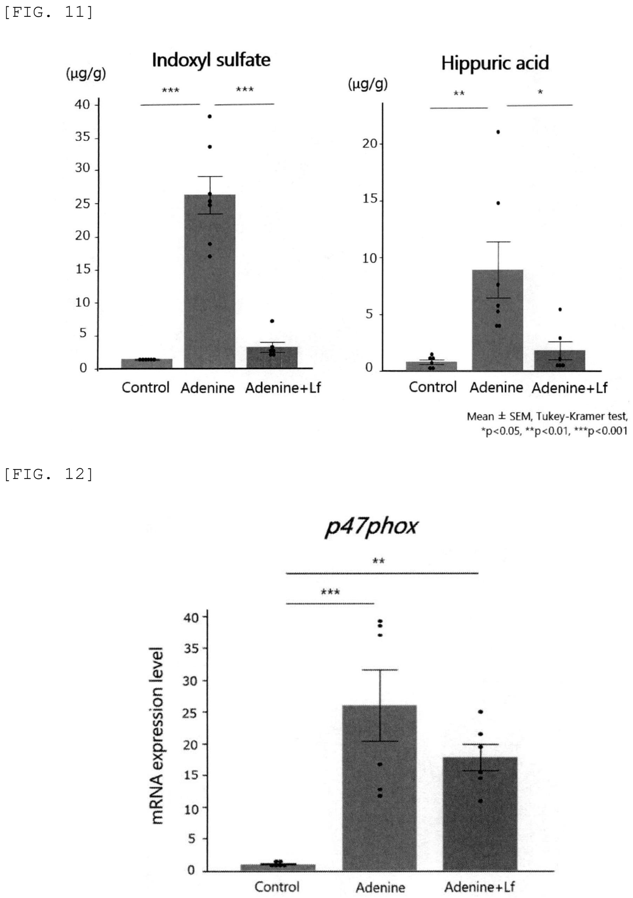

The concentrations of indoxyl sulfate and hippuric acid in the muscle are shown in FIG. 11. From the results shown in FIG. 11, it was found that lactoferrin inhibits uremic toxin accumulation in the muscle caused by adenine administration.

From the above results, it was found that the progression of the complications of chronic kidney disease was inhibited in the lactoferrin administration group (Adenine + Lf administration group) compared to the renal failure group (Adenine administration group). Accordingly, lactoferrin can be used for prevention of a complication of chronic kidney disease.

[No Influence on Adenine Administration-Caused Oxidative Stress in Kidneys by Lactoferrin]

<RNA Extraction and Reverse Transcription>

The kidneys collected during the dissection of the mice were frozen rapidly with liquid nitrogen and then stored at -80°C until RNA extraction. Each kidney was homogenized in 1.0 mL of TRI Reagent (#TR 118; Molecular Research Center, Inc. Ohio, USA), and the homogenate was transferred to a 1.5-mL tube. To the homogenate, 200 μL of chloroform was added, and the mixture was vortexed for 20 seconds. After leaving still at room temperature for two to three minutes, the mixture was centrifuged at 12,000× g at 4°C for 15 minutes. The upper layer (aqueous layer) was transferred to a new 1.5-mL tube, and 500 μL of 2-propanol was added. After mixing by inversion, the mixture was left still at room temperature for 10 minutes. Then, after centrifugation at 12,000× g at 4°C for 15 minutes, the supernatant was discarded, and 1.0 mL of 75% ethanol was added. After centrifugation at 7,500× g at 4°C for five minutes after vortexing, the supernatant was discarded, and the resultant was dried at room temperature. Then, 50 mL of TE buffer was added, and the pellet was dissolved by tapping. The yield and the purity of the total RNA were determined by measuring the absorbances at 260 nm and 280 nm using NanoDrop 2000 (Thermo Fisher Scientific, Inc.). The extracted total RNA was stored at -80°C until reverse transcription.

For the reverse transcription, iScript Advanced cDNA Synthesis Kit for RT-PCR (Bio-Rad) was used according to the instruction manual. A premix in a volume of 2.5 μL obtained by mixing iScript advanced reverse transcriptase and 5× iScript advanced reaction mix was added to 2.5 μg of the total RNA, and the volume was adjusted to total 10 μL with sterilized dH$_2$O. The reverse transcription was conducted with T100 (registered trademark) Thermal cycler (Bio-Rad). The PCR program was as follows.

Step 1: 42°C for 30 min
Step 2: 85°C for 5 min
Step 3: 4°C for 0 min
END

The composition of the premix is shown in Table 1.

[Table 1]

|  | for 1 sample |
| --- | --- |
| 5 x iScript advanced reaction mix | 2 μL |
| iScript advanced reverse transcriptase | 0.5 μL |

<Real-Time PCR>

The cDNA solution was diluted 50 folds with MilliQ water and used for analysis. To each well of a PCR 96-well plate (Hard-Shell 96-Well Skirted PCR Plates; Bio-rad), 2 μL of the cDNA solution and 10 μL of a premix were added, and real-time PCR was conducted using a CFX Connect (registered trademark) real-time PCR analysis system (Bio-Rad) with the program shown below.

Step 1: 95°C for 60 sec
Step 2: 95°C for 5 sec
Step 3: 60°C for 15 sec
Plate Read
Step 4: Go to Step 2 39 times
END

Luna Universal qPCR Master Mix (BioLabs, #M3003E, Lot. 10061403), which uses an equivalent dye to SYBR Green and which can be detected with a SYBR/FAM filter, was used according to the instruction manual. The composition of the premix is shown in Table 2 below, and the primers used are shown in Table 3 below. Here, the primers used were purchased from Takara Bio Inc. (the primer set IDs in Table 3 are the model numbers). For the quantification of the relative mRNA amounts in the organs, the calibration curve method was used. β-actin (Actb) was used as the housekeeping gene of the samples, and the expression levels were indicated with the relative values based on the expression level of the Control group regarded as 1.

[Table 2]

|  | for 1 sample | Final concentration |
| --- | --- | --- |
| Luna Universal qPCR Master Mix | 5 μL | 1x |
| 10 μM Primer (forward) | 0.25 μL | 250 nM |
| 10 μM Primer (reverse) | 0.25 μL | 250 nM |
| RNase-free H$_2$O | 4.5 μL | - |
| Total | 10 μL | - |

[Table 3]

| Gene | Species | Primer Set ID |
| --- | --- | --- |
| *p47 phox* | mouse | MA175153 |
| *Actb* | mouse | MA050368 |

The results are shown in FIG. 12. p47phox is an important component of NADPH oxidase, and as the expression of p47phox becomes higher, the oxidative stress in the kidneys becomes larger. From the results shown in FIG. 12, oxidative stress was increased in the kidneys by adenine administration, but the administration of lactoferrin did not inhibit the oxidative stress with significance. That is, this means that, although it was suggested that lactoferrin inhibits symptoms of chronic kidney disease (complications) caused by adenine administration in various types of experiment in the present application, the mechanism of action of inhibiting chronic kidney disease (complications) is not inhibition of the oxidative stress in the kidneys.

On the other hand, NPL 1 discloses that, while the survival rate of cells decreases through administration of hydrogen peroxide, the survival rate of cells improves when lactoferrin is administered. That is, in NPL 1, lactoferrin inhibits apoptosis through hydrogen peroxide, in other words, lactoferrin inhibits oxidative stress and thus inhibits renal damage. Then, this means that the mechanism of action of lactoferrin shown in FIG. 12 and the mechanism of action of lactoferrin described in NPL 1 are different mechanisms. That is, the action is on damage without oxidative stress.

As shown above, from the adenine-induced renal failure model mice used in the present application and the model mice described in NPL 1, which shift to chronic kidney disease from acute kidney injury through induction with folic acid, different results were obtained even when the same substance was administered. Accordingly, it was found that the mechanism of action specific to chronic kidney disease cannot be predicted from experiment using model mice of acute kidney injury.

[Change in Metabolites in Muscle]

To examine the changes in metabolism through adenine administration and lactoferrin administration, metabolites were measured using quadriceps femoris muscle obtained during the dissection. The measurement was conducted by the following procedures.

(1) Pretreatment

The weights of the left quadriceps femoris muscles of the mice which were stored at -80°C were measured, and the muscles were homogenized in a pretreatment solution in a volume of tissue amount (g) $\times$ 1000 $\times$ 10 ($\mu$L). The composition of the pretreatment solution is as follows.

In a mixture solution of $H_2O$:methanol:$CHCl_3$ = 2:5:2, 1/10 the amount of 0.5 mg/mL isopropyl malic acid (333115-100MG; ALDRICH) was dissolved as an internal standard substance.

After shaking with heating at 1200 rpm at 37°C for 30 minutes, centrifugation was conducted at 16,000$\times$ g at 4°C for three minutes, and 200 $\mu$L of the supernatant was collected. After adding 200 $\mu$L of ultrapure water and vortexing, centrifugation was conducted at 16,000$\times$ g at 4°C for three minutes, and 250 $\mu$L of the supernatant was collected. After the solvent was removed using CENTRIFUGAL EVAPORATOR CVE-3100 (EYELA, Tokyo, Japan) and UNITRAP UT-2000 (EYELA), the resultant was frozen at -80°C and freeze-dried with CENTRIFUGAL EVAPORATOR CVE-1000 (EYELA) and FREEZE DRYER FDU-830 (EYELA). To the freeze-dried sample, 80 $\mu$L of 20 mg/mL methoxyamine hydrochloride (#136-05933; WAKO) pyridine solution was added, and the pellet was dissolved by vortexing and pipetting. After ultrasonic treatment for 20 minutes and shaking at 1,200 rpm at 30°C for 90 minutes, 40 $\mu$L of N-methyl-N-trimethylsilyl-trifluoroacetamide (MSTFA) (#1022-11061; GL Sciences Inc.) was added, and the mixture was shaken at 1,200 rpm at 37°C for 30 minutes. After centrifugation at 16,000$\times$ g for three minutes, 80 $\mu$L of the supernatant was transferred to a vial (#227-34120-01; shimadzu) and used as a measurement sample. QC was created by collecting 10 $\mu$L from each sample.

(2) Measurement

The measurement conditions of GC-MS are shown in Table 4. As the metabolites, 481 metabolites were analyzed using the retention times described in Smart Metabolites Database (Shimadzu Co.). The results were indicated with the relative values based on the average of the control group regarded as 1.

[Table 4]

| MS system | GCMS-QP2010 Plus (Shimadzu Co.) |
|---|---|
| Analytical column | (30 m $\times$ 0.25 mm inner diameter, film thickness: 0.25 $\mu$m; Shimadzu Co.) |
| Mobile phase | Helium gas |
| Flow rate | 39.0 cm/sec |
| front inlet temperature | 250°C |
| interface temperature | 280°C |
| ion-source temperature | 200°C |

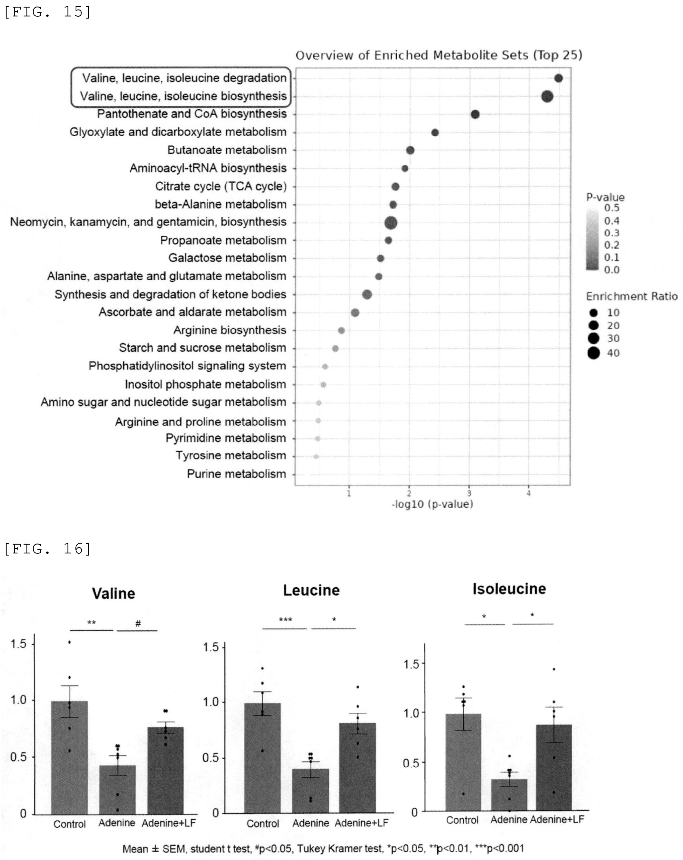

Based on the 104 detected metabolites, enrichment analysis was conducted with MetaboAnalyst 5.0. The results are shown in FIG. 15. As shown in FIG. 15, significant changes were observed in the degradation of valine, leucine and isoleucine (the three amino acids are sometimes together referred to as "BCAA" below) and in the biosynthesis of BCAA.

The results of the comparison of the valine, leucine and isoleucine amounts in the quadriceps femoris muscle among the three groups are shown in FIG. 16. As it is obvious from FIG. 16, BCAA all decreased with significance in the Adenine group compared to those of the Control group and increased with significance in the Adenine + LF group compared to those of the Adenine group. From the above results, it was found that lactoferrin increases BCAA in the muscle.

BCAA are amino acids which are highly related to the protein metabolism of the skeletal muscles, and it is known that the BCAA concentrations in the cells decrease in CKD patients (Noel J. M. Cano et al., "Application of Branched-Chain Amino Acids in Human Pathological States: Renal Failure", J Nutr., 2006, Jan; 136 (1 Suppl): 299S-307S. doi: 10.1093/jn/136.1.299S.). From the results shown in FIG. 16, it is believed that lactoferrin administration has mechanism of action of inhibiting progression of sarcopenia through inhibition of the decrease in BCAA.

[Analysis of Muscular Protein Expression]

To examine the involvement of LF in the synthetic pathways of proteins and autophagy in the muscles, quantification of p70S6K, phospho-p70S6K, AMPK, phospho-AMPK, LC3, β-actin and cathepsin proteins was conducted by the following procedures.

(1) Sample Production

By mixing 10× RIPA (9806s; Cell Signaling Technology, Inc, Danvers, USA), 100 mM PMSF (36978; Thermo Fisher Scientific, San Jose, CA, USA), a protease inhibitor cocktail (11836153001; Sigma-Aldrich, Co., St. Louis, MO, USA), a phosphatase inhibitor (P5726; Sigma-Aldrich, Co., St. Louis, MO, USA), MG-132 (M7449; Sigma-Aldrich, Co., St. Louis, MO, USA) and ultrapure water, 1× RIPA buffer was prepared. The composition of the RIPA buffer is shown in Table 5.

[Table 5]

|  | For 100 mg sample |
| --- | --- |
| 10× RIPA | 100 μL |
| 100 mM PMSF | 10 μL |
| Protease Inhibitor Cocktail | 150 μL |
| Phosphatase Inhibitor | 1 μL |
| MG-132 | 1 μL |
| Ultrapure Water | 738 μL |
| Total | 1000 μL |

The weight of each mouse skeletal muscle sample was measured, and the sample was put into a 2-mL tube. According to the sample weight, 1× RIPA buffer, which was prepared in advance, was added (1 mL/100 mg sample), and the tissue was homogenized using Polytron PT 1300 D (Kinematica AG, Malters, Switzerland) on ice. Then, centrifugation was conducted at 14,000× g for 10 minutes, and the supernatant was collected.

(2) Quantification of Extracted Protein

The extracted protein was diluted 20 folds with ultrapure water. To 500 μL of Quick Start (registered trademark) Bradford 1× Dye Reagent (500-0205; Bio-Rad Laboratories, Inc., Hercules, CA, USA), 10 μL of BSA Standard (5000207; Bio-Rad Laboratories, Inc., Hercules, CA, USA) or the 20-fold-diluted sample was added and mixed. To a 96-well plate, 200 μL of the mixture solutions were applied. Then, after the absorbances at a measurement wavelength of 595 nm were measured with Spectra Max190 (Molecular Devices Washington, D.C., USA), a calibration curve was created, and the protein concentration of the sample was calculated.

(3) Western Blotting

2× Laemmli Sample Buffer (161-0747; Bio-Rad Laboratories, Inc., Hercules, CA, USA) and 2-mercaptoethanol (161-6710; Bio-Rad Laboratories, Inc., Hercules, CA, USA) were mixed, and thus a loading buffer was prepared. From the results of the protein quantification, the sample amount and the necessary amounts of the loading buffer and ultrapure water were calculated, and after mixing, heat blocking was conducted at 95°C for four minutes.

For electrophoresis, a gel produced with TGX™ FastCast (registered trademark) Acrylamide Kit, 12% (161-0175; Bio-Rad Laboratories, Inc., Hercules, CA, USA) was used. The proteins were transferred to a membrane of Trans-Blot Turbo Mini Format 0.2 μm, PVDF (1704156; Bio-Rad Laboratories, Inc., Hercules, CA, USA). Primary antibodies which were diluted 1000 folds with Solution 1 (NKB-101; Toyobo, Co, Ltd., Osaka, Japan) were added and enclosed, and the membrane was shaken at 4°C overnight. On the next day, the membrane was taken out of the hybridization bag and washed with TBST-20, and the membrane was put into a new hybridization bag. Secondary antibodies which were diluted 5000 folds with Solution 2 (NKB-101; Toyobo, Co, Ltd., Osaka, Japan) were added and enclosed, and the membrane was

**EP 4 674 428 A1**

shaken at room temperature for one hour and then washed with TBST-20. After 0.5 ml of the reagent of Super Signal West Dura Extended Duration Substrate (rr34075; Thermo Fisher Scientific, San Jose, CA, USA) was placed on the membrane and reacted for one minute, an image was taken with Chemi Doc MP (Bio-Rad Laboratories, Inc., Hercules, CA, USA). The primary antibodies and the secondary antibodies are shown in Table 6. The obtained bands were quantified using Image Lab 6.0.1 (Bio-Rad Laboratories, Inc., Hercules, CA, USA). * Composition of TBST-20

TBST-20 was produced by mixing at a ratio of 10× TBS (64573292; Bio-Rad):ultrapure water:polyoxyethylene(20) sorbitan monolaurate (166-21213; FUJIFILM Wako Pure Chemical Corporation) = 250:2250:2.

[Table 6]

|  | Antigen | Manufacturer | Cat. No. | Host | Dilution Rate |
|---|---|---|---|---|---|
| Primary Antibody | p70S6K | Cell Signaling | 9202 | Rabbit | 1000 |
|  | phospho-p70S6K (Thr389) |  | 9205 |  |  |
|  | LC3A/B (D3U4C) XP® |  | 12741 |  | 2000 |
|  | AMPK |  | 2532 |  | 1000 |
|  | phospho-AMPK |  | 2531 |  |  |
|  | Cathepsin B |  | 31718 |  |  |
|  | β-actin | Santa Cruz Biotechnology | sc-47778 |  |  |
| Secondary Antibody | anti-rabbit IgG-HRP |  | sc-2357 | Mouse | 5000 |
|  | anti-mouse IgG-HRP |  | sc-2031 | goat |  |

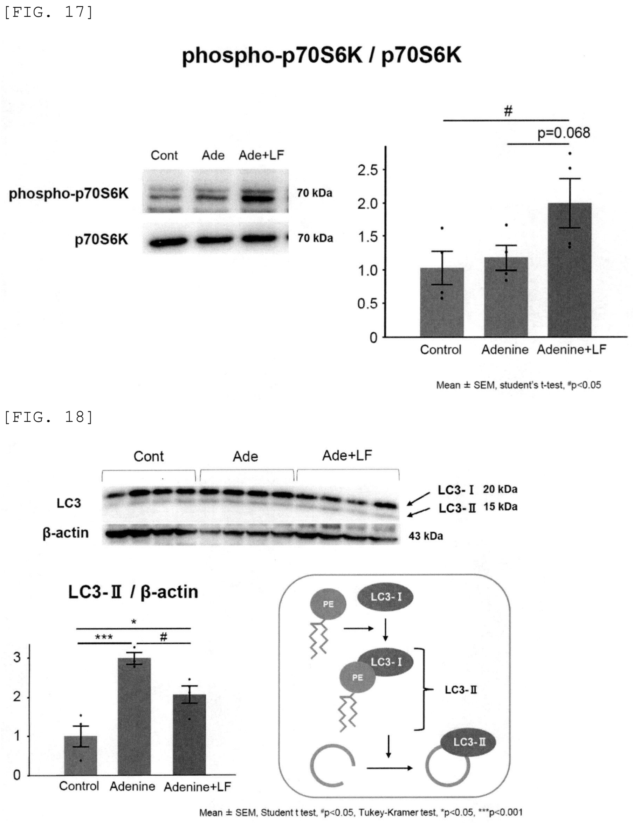

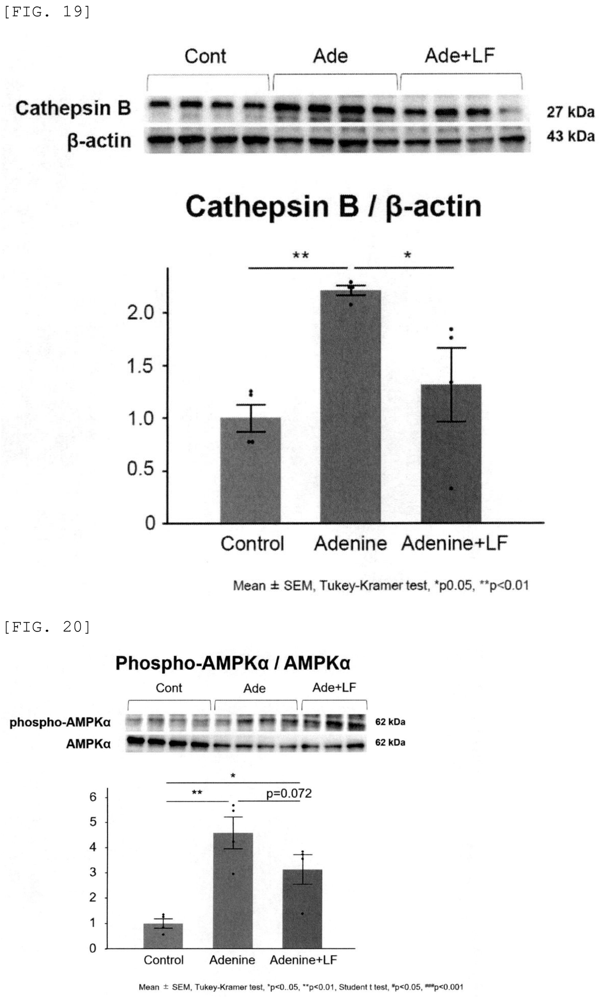

In FIG. 17, the pictures of western blotting of phospho-p70S6K (phosphorylated) and p70S6K (not phosphorylated) in the quadriceps femoris muscle and the results of the comparison of the phosphorylation rates among the three groups, where the phosphorylated p70S6K amounts relative to the expression levels of non-phosphorylated p70S6K were quantified by quantifying the band intensities and the quantified values were compared based on that of the Control regarded as 1, are shown. In FIG. 18, the pictures of western blotting of LC3A/B and β-actin in the quadriceps femoris muscle and a graph showing the comparison of the expression levels among the three groups, where the expression levels of LC3-II were corrected with β-actin and the quantified values were compared based on that of the Control regarded as 1, are shown. In FIG. 19, the pictures of western blotting of cathepsin B and β-actin in the quadriceps femoris muscle and a graph showing the comparison of the expression levels among the three groups, where the expression levels of cathepsin B were corrected with β-actin and the quantified values were compared based on that of the Control regarded as 1, are shown. In FIG. 20, the pictures of western blotting of phospho-AMPKα (phosphorylated) and AMPKα (not phosphorylated) in the quadriceps femoris muscle and the results of the comparison of the phosphorylation rates among the three groups, where the phosphorylated AMPKα amounts relative to the expression levels of non-phosphorylated AMPKα were quantified by quantifying the band intensities and the quantified values were compared based on that of the Control regarded as 1, are shown.

As shown in FIG. 17, the phosphorylation of p70S6K of the Adenine + LF group was accelerated with significance compared to that of the Control group and tended to be accelerated also compared to that of the Adenine group. Moreover, as shown in FIG. 18, although two bands of LC3-I and LC3-II were observed in the western blotting analysis of LC3, the expression of LC3-II, which is used as an autophagosome marker, increased in the Adenine group, and the increase was inhibited with significance in the Adenine + LF group. Moreover, as shown in FIG. 19, the expression of cathepsin B, which is a characteristic hydrolase of lysosomes, increased in the Adenine group, and the increase was significantly inhibited by the LF administration.

It is known that p70S6K, which is a protein downstream of the mTOR signaling cascade, involves in promotion of synthesis by increasing the translation efficiency of protein. From the results shown in FIG. 17, because phosphorylation of p70S6K was accelerated through the administration of lactoferrin, it is believed that lactoferrin promoted the synthesis of muscular protein.

In the muscles of the CKD model mice, autophagy is accelerated. Excessive autophagy causes muscle atrophy. As shown in FIG. 18 and FIG. 19, it is believed that the expression of LC3-II and cathepsin B, which are proteins that accelerate autophagy, was inhibited when lactoferrin was administered and that muscle atrophy was thus inhibited.

Activation of AMPK induces acceleration of autophagy. Although phosphorylation of AMPKα increased in the Adenine group, the increase was inhibited in the Adenine + LF group. From the results, it is believed that the LF administration inhibited deterioration of the pathological condition and thus inhibited phosphorylation of AMPKα and that, as a result, the increases in the expression of LC3-II, which is a marker for formation of autophagosomes that are in the early stage of autophagy, and cathepsin B, which is a characteristic enzyme in degradation in lysosomes, were inhibited. From the results, it is believed that the inhibition of protein degradation by autophagy involved in the inhibition of progression of sarcopenia and that the LF administration inhibited protein degradation by autophagy.

[Experimental Animals and Experiment Procedures: Examination of Therapeutic Effects on Complications of Chronic Kidney Disease]

By the same procedures as those in [Experimental Animals and Experiment Procedures: Examination of Prevention Effects on Complications of Chronic Kidney Disease] above, mice of a renal failure group (Adenine administration group) and a control group (Control group) were prepared. As it is obvious from the experiment in [Experimental Animals and Experiment Procedures: Examination of Prevention Effects on Complications of Chronic Kidney Disease] above, the renal failure group developed complications of chronic kidney disease.

Next, the prepared renal failure group was divided randomly into a group without lactoferrin administration (indicated by "Adenine" in the figures described below) and a lactoferrin administration group (indicated by "Adenine + Lf" in the figures described below) and fed as follows.

A control MF food was given to the renal failure group and the control group for four weeks. Moreover, tap water was given by free drinking.

The control MF food was given to the lactoferrin administration group for four weeks. Moreover, an aqueous 2% lactoferrin solution was given by free drinking instead of tap water.

After four weeks passed, samples were collected from the mice, and various types of analysis were conducted by the same procedures as those in [Experimental Animals and Experiment Procedures: Examination of Prevention Effects on Complications of Chronic Kidney Disease] above.

[Treatment of Renal Atrophy and Heart Hypertrophy by Lactoferrin]

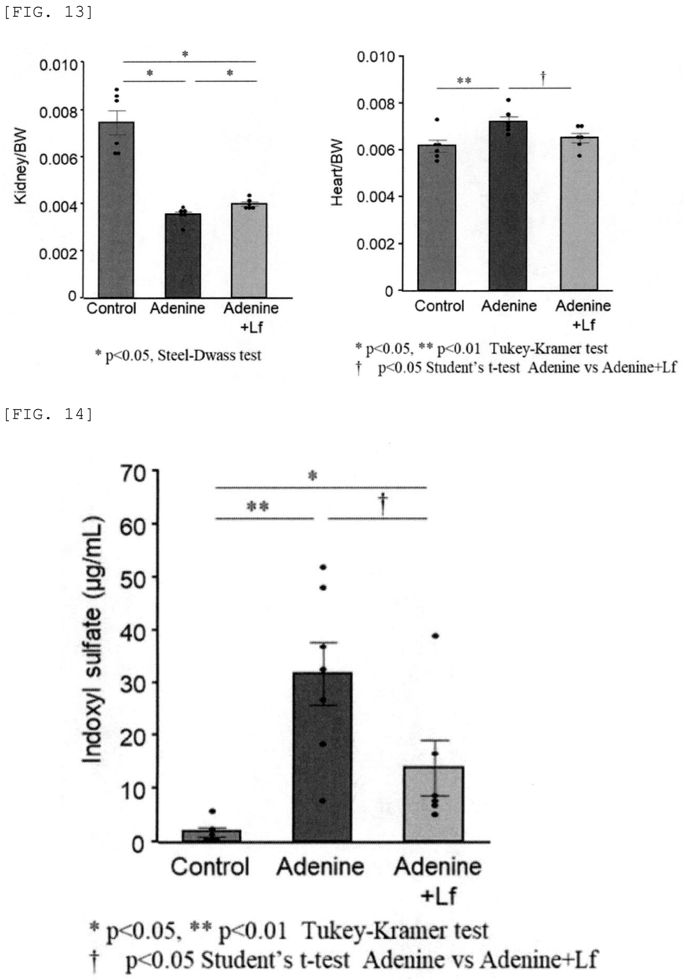

A graph of the kidney weights corrected with the body weights is shown on the left in FIG. 13, and a graph of the heart weights corrected with the body weights is shown on the right. As it is obvious from FIG. 13, the kidney weight decreased and the heart weight increased in the group without lactoferrin administration (Adenine) and the lactoferrin administration group (Adenine + Lf) compared to those of the control group (Control). On the other hand, the kidney weight increased with significance and the heart weight decreased in the lactoferrin administration group (Adenine + Lf) compared to those of the group without lactoferrin administration (Adenine). That is, it was found that renal atrophy is improved and heart hypertrophy is inhibited when lactoferrin is administered to a mouse which has developed renal failure.

[Inhibition of Increase in Blood Uremic Toxin by Lactoferrin]

The blood indoxyl sulfate concentration increased with significance in the group without lactoferrin administration (Adenine) and the lactoferrin administration group (Adenine + Lf) compared to that of the control group (Control). On the other hand, the blood indoxyl sulfate concentration decreased with significance in the lactoferrin administration group (Adenine + Lf) compared to that of the group without lactoferrin administration (Adenine). That is, it was found that the blood uremic toxin amount decreases when lactoferrin is administered to a mouse which has developed renal failure.

From the above results, therapeutic effects on the complications of chronic kidney disease were observed in the lactoferrin administration group (Adenine + Lf) compared to the renal failure group (Adenine) which developed the same renal failure. Accordingly, lactoferrin can be used for treatment of a complication of chronic kidney disease.

[Examination of Therapeutic Effects on Advanced Chronic Kidney Disease]

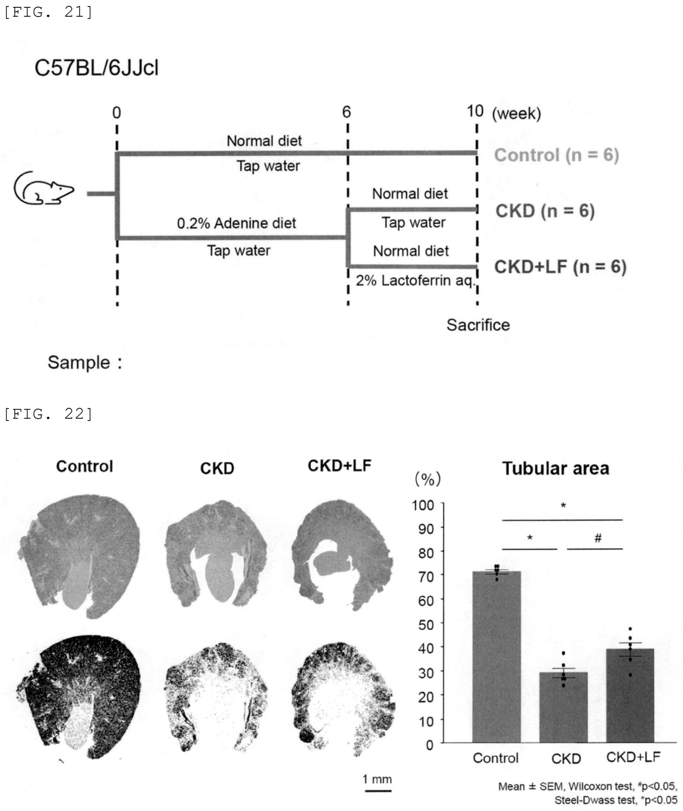

Referring to FIG. 21, the mice used in the experiment are explained. In the experiment, a renal failure group (n = 12) which was obtained by the same procedures to those of the renal failure group (Adenine administration group) mice in [Experimental Animals and Experiment Procedures: Examination of Prevention Effects on Complications of Chronic Kidney Disease] above was divided into two groups. To one of the groups, a control MF food containing no adenine was given for four weeks, and tap water was given by free drinking (CKD group). To the other group, the control MF food containing no adenine was given for four weeks, and an aqueous 2% lactoferrin solution was given by free drinking instead of tap water (the CKD + LF group in FIG. 21). To the control group, the control MF food containing no adenine was given for 10 weeks, and tap water was given by free drinking.

By the experiment shown below, the effects of lactoferrin administration on CKD mice obtained by administering adenine for six weeks were examined. In this regard, for the convenience, the experiment in [Experimental Animals and Experiment Procedures: Examination of Prevention Effects on Complications of Chronic Kidney Disease] above is sometimes referred to as "Example 1".

[Renal Histologic Analysis]

The experiment was conducted by the same procedures as those in Example 1. In FIG. 22, pathological pictures of the Masson trichrome-stained kidneys of the groups and a graph showing the comparison of the quantified remaining renal tubule rates among the three groups are shown. As it is obvious from FIG. 22, the ratio of the remaining renal tubular area in the cortex area decreased with significance in the CKD group and the CKD + LF group compared to that of the Control group but increased with significance in the CKD + LF group compared to that of the CKD group. From the above results, it was found that lactoferrin can treat the tubulointerstitial disease of CKD.

[Gene Expression Analysis]

By the same procedures as those in <RNA Extraction and Reverse Transcription> in Example 1, the gene expression of Tgfb1, Ctgf, Pail, Col1a1, Col3a1 and Col4a1, which are fibrogenesis-related genes, in the kidneys was analyzed. The expression levels were corrected with Gapdh. In this regard, of the primers used, those purchased from a manufacturer (Takara Bio Inc.) are shown in Table 7 with the model numbers. The synthesized primers are shown in Table 8 with the sequences.

[Table 7]

| Gene | Species | Set ID |
|------|---------|--------|
| Gapdh | mouse | MA050371 |
| Ctgf | mouse | MA028643 |
| Tgfb1 | mouse | MA148599 |

[Table 8]

| Gene | Species | Type | Sequences (5'-3') | SEQ.ID |
|------|---------|------|-------------------|--------|
| Col1a1 | mouse | Forward | TGACTGGAAGAGCGGAGAGT | 1 |
| Col1a1 | mouse | Reverse | GAATCCATCGGTCATGCTCT | 2 |
| Col3a1 | mouse | Forward | GTGAAACTGGTGAACGTGGCTCTA | 3 |
| Col3a1 | mouse | Reverse | AGGACCTGGATGCCCACTTG | 4 |
| Col4a1 | mouse | Forward | GGCTATTCCTTCGTGATGCA | 5 |
| Col4a1 | mouse | Reverse | CTAAACTCTTCCAGACAGGAC | 6 |
| Pai1 (Serpine1) | mouse | Forward | TTCAGTGGCCAATGGAAGACTCCT | 7 |
| Pai1 (Serpine1) | mouse | Reverse | AGGGCAGTTCCACAACGTCATACT | 8 |

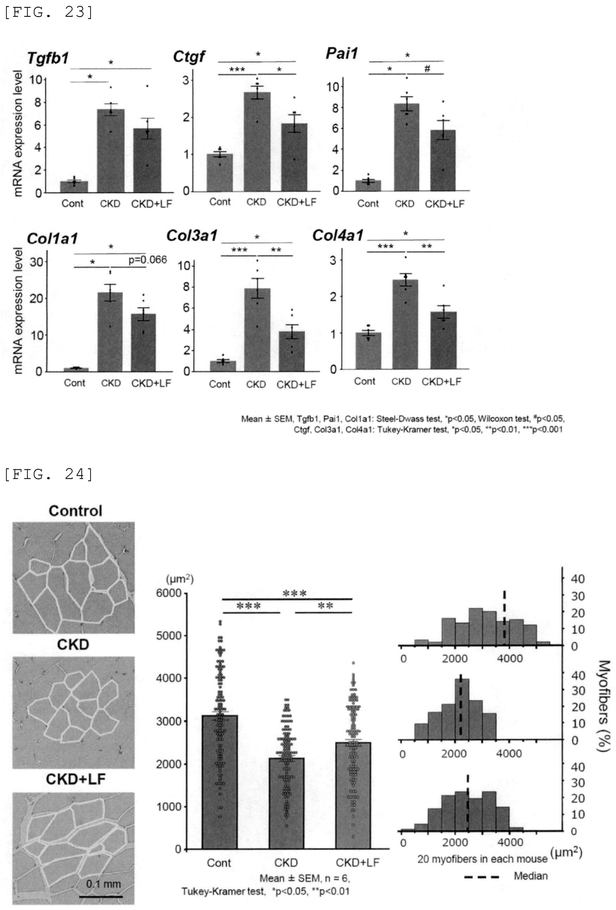

In FIG. 23, the results of the comparison of the expression levels of the fibrogenesis-related genes in the kidneys among the three groups are shown. As it is obvious from FIG. 23, the expression of all the genes of Tgfb1, Ctgf, Pail, Col1a1, Col3a1 and Col4a1, which are fibrogenesis-related genes, in the kidneys increased with significance in the CKD group and the CKD + LF group compared to that of the Control group. Moreover, a tendency towards a decrease in the expression of Col1a1 and a significant decrease in the expression of Col3a1, Col4a1, Ctgf and Pai1 were observed in the CKD + LF group compared to those of the CKD group. From the above results, it was found that administration of lactoferrin to CKD exerts an anti-fibrogenesis action through a decrease in the expression of fibrogenesis-related genes.

[Treatment of Sarcopenia of CKD by Lactoferrin]

<Evaluation of Muscle Cross Section>

The experiment was conducted by the same procedures as those in <Evaluation of Muscle Cross Section> of Example 1. Pictures of typical cross sections of H&E-stained quadriceps femoris muscles, a graph showing the comparison of the quantified muscle cross sectional areas among the three groups and the distributions of the muscle cross sectional areas are shown in FIG. 24. As it is obvious from FIG. 24, it was found that lactoferrin administration improves the muscle atrophied with CKD.

<Indoxyl Sulfate Concentration in Muscle>

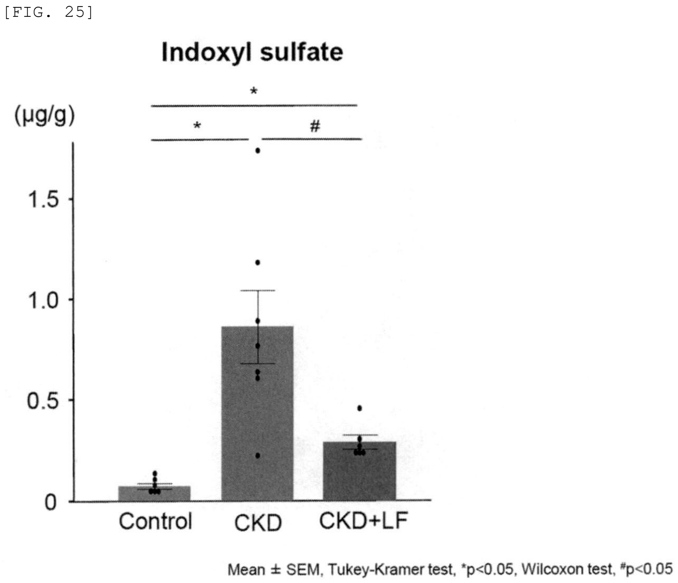

The indoxyl sulfate concentrations in the muscle of quadriceps femoris muscle were examined by the same procedures to those of [Inhibition of Adenine Administration-Caused Sarcopenia by Lactoferrin] of Example 1. The results of the comparison among the three groups are shown in FIG. 25. As it is obvious from FIG. 25, it was found that lactoferrin administration improves accumulation of the uremic toxin in the muscle.

From the above results, it was found that lactoferrin exerts a therapeutic effect on advanced chronic kidney disease.

Industrial Applicability

The composition disclosed in the present application can be used for treatment and/or prevention of a complication of chronic kidney disease. Moreover, with the inhibitor of progression of renal damage, the inhibitor of progression of sarcopenia and the inhibitor of progression of anemia, progression of renal damage, sarcopenia and anemia, which are complications of chronic kidney disease, can be prevented, or the progression can be inhibited. Thus, it is useful for the medical industry.

**Claims**

1. A composition for use in treatment and/or prevention of a complication of chronic kidney disease, comprising lactoferrin as an active ingredient.

2. The composition according to claim 1,
   wherein the treatment and/or the prevention of the complication of chronic kidney disease is inhibition of progression of sarcopenia and/or anemia through inhibition of progression of renal damage in chronic kidney disease.

3. The composition according to claim 2,
   wherein the inhibition of progression of renal damage is inhibition of progression of at least one selected from the group consisting of renal atrophy, a tubulointerstitial disease and renal impairment.

4. The composition according to claim 2,
   wherein the inhibition of progression of renal damage is inhibition of renal inflammation and/or fibrogenesis.

5. The composition according to claim 2,
   wherein the inhibition of progression of sarcopenia is inhibition of reduction in a muscle bundle section and/or a decrease in uremic toxin accumulation in a muscle.

6. An inhibitor of progression of renal damage in chronic kidney disease, comprising lactoferrin as an active ingredient.

7. An inhibitor of progression of sarcopenia in chronic kidney disease, comprising lactoferrin as an active ingredient.

8. An inhibitor of progression of anemia in chronic kidney disease, comprising lactoferrin as an active ingredient.

9. The composition according to any one of claims 1 to 5 which is used as a component of a food composition or a component of a pharmaceutical composition.

10. The inhibitor of progression according to any one of claims 6 to 8 which is used as a component of a food composition or a component of a pharmaceutical composition.

[FIG. 1]

A

B

Mean ± SEM, Tukey-Kramer test,
vs Control, *p<0.05, **p<0.01, ***p<0.001, vs Adenine, #p<0.05, ###p<0.001

[FIG. 2]

KIDNEY WEIGHT/BODY WEIGHT

Mean ± SEM, Tukey-Kramer test, *p<0.05

[FIG. 3]

**BUN**

Mean ± SEM, Tukey-Kramer test, vs Control, **p<0.01, ***p<0.001
vs Adenine, ###p<0.001

[FIG. 4]

**BLOOD CREATININE**

Mean ± SEM, Tukey-Kramer test, ***p<0.001

[FIG. 5]

Mean ± SEM, Tukey-Kramer test, *p<0.05, ***p<0.001

[FIG. 6]

Mean ± SEM, Tukey-Kramer test, **p<0.01, ***p<0.001

[FIG. 7]

## HE STAINING

[FIG. 8]

[FIG. 9]

HEMOGLOBIN

HEMATOCRIT

Mean ± SEM, Tukey-Kramer test,, vs Control, **p<0.01, ***p<0.001, vs Adenine, ###p<0.001

[FIG. 10]

MUSCLE BUNDLE SECTIONAL AREA
(FEMORIS MUSCLE)

Mean ± SEM, n = 6,
Tukey-Kramer test,
*p<0.05, **p<0.01, ***p<0.001

20 myofibers in each mouse
--- Median

[FIG. 11]

Mean ± SEM, Tukey-Kramer test,
*p<0.05, **p<0.01, ***p<0.001

[FIG. 12]

[FIG. 13]

* p<0.05, Steel-Dwass test

* p<0.05, ** p<0.01 Tukey-Kramer test
† p<0.05 Student's t-test Adenine vs Adenine+Lf

[FIG. 14]

* p<0.05, ** p<0.01 Tukey-Kramer test
† p<0.05 Student's t-test Adenine vs Adenine+Lf

[FIG. 15]

Overview of Enriched Metabolite Sets (Top 25)

[FIG. 16]

Mean ± SEM, student t test, #p<0.05, Tukey Kramer test, *p<0.05, **p<0.01, ***p<0.001

[FIG. 17]

## phospho-p70S6K / p70S6K

Mean ± SEM, student's t-test, #p<0.05

[FIG. 18]

### LC3-II / β-actin

Mean ± SEM, Student t test, #p<0.05, Tukey-Kramer test, *p<0.05, ***p<0.001

[FIG. 19]

Cont    Ade    Ade+LF

Cathepsin B    27 kDa
β-actin    43 kDa

## Cathepsin B / β-actin

Control    Adenine    Adenine+LF

Mean ± SEM, Tukey-Kramer test, *p0.05, **p<0.01

[FIG. 20]

## Phospho-AMPKα / AMPKα

Cont    Ade    Ade+LF

phospho-AMPKα    62 kDa
AMPKα    62 kDa

Control    Adenine    Adenine+LF

p=0.072

Mean ± SEM, Tukey-Kramer test, *p<0..05, **p<0.01, Student t test, #p<0.05, ###p<0.001

[FIG. 21]

[FIG. 22]

[FIG. 23]

Mean ± SEM, Tgfb1, Pai1, Col1a1: Steel-Dwass test, *p<0.05, Wilcoxon test, #p<0.05, Ctgf, Col3a1, Col4a1: Tukey-Kramer test, *p<0.05, **p<0.01, ***p<0.001

[FIG. 24]

Mean ± SEM, n = 6, Tukey-Kramer test, *p<0.05, **p<0.01

[FIG. 25]

**Indoxyl sulfate**

Mean ± SEM, Tukey-Kramer test, *p<0.05, Wilcoxon test, #p<0.05

## INTERNATIONAL SEARCH REPORT

International application No.

**PCT/JP2024/017376**

### A. CLASSIFICATION OF SUBJECT MATTER

*A61K 38/40*(2006.01)i; *A23L 33/19*(2016.01)i; *A61P 7/06*(2006.01)i; *A61P 13/12*(2006.01)i; *A61P 21/00*(2006.01)i
FI: A61K38/40; A61P13/12; A61P21/00; A61P7/06; A23L33/19

According to International Patent Classification (IPC) or to both national classification and IPC

### B. FIELDS SEARCHED

Minimum documentation searched (classification system followed by classification symbols)

A61K38/40; A23L33/19; A61P7/06; A61P13/12; A61P21/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
|---|---|---|
| X | KASHYAP, M. et al., WCN23-0108 Effectiveness of Lactoferrin in the Treatment of Anemia in Chronic Kidney Disease: A Single Center Pilot Study Evidence, Kidney International Reports, March 2023, vol. 8, no. 3, page S133, DOI: 10.1016/j.ekir.2023.02.302 particularly, sections of introduction, conclusion | 1-4, 8-10 |
| Y | | 1-10 |
| X | JP 2014-227375 A (MORINAGA MILK INDUSTRY CO., LTD.) 08 December 2014 (2014-12-08) claims 1, 2, paragraph [0065], tables 1, 2, 5, 7 | 1, 6, 9, 10 |
| Y | | 1-10 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **18 June 2024** | **02 July 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

## INTERNATIONAL SEARCH REPORT

| International application No. |
|---|
| **PCT/JP2024/017376** |

### C. DOCUMENTS CONSIDERED TO BE RELEVANT

| Category* | Citation of document, with indication, where appropriate, of the relevant passages | Relevant to claim No. |
|---|---|---|
| X | 福島萌, 他2名, 慢性腎不全モデルラットにおけるラクトフェリンの腎保護効果, 日本獣医学会学術集会講演要旨集, 2014, vol. 157, page 489, HSO-49, non-official translation (FUKUSHIMA, Moe and 2 others, Renal protective effect of lactoferrin in chronic renal failure model rats, Abstracts of the Meeting of the Japanese Society of Veterinary Science) 特に、背景と目的の項、結果および考察の項, non-official translation (particularly, section of background and objectives, section of results and discussion) | 1, 6, 9, 10 |
| Y | | 1-10 |
| X | HSU, Y. H. et al., Lactoferrin Contributes a Renoprotective Effect in Acute Kidney Injury and Early Renal Fibrosis, Pharmaceutics, 2020, vol. 12, no. 5, Article No. 434, DOI: 10.3390/pharmaceutics12050434 abstract | 1, 6, 9, 10 |
| Y | | 1-10 |
| Y | GONZALEZ, P. et al., Unraveling the Metabolic Hallmarks for the Optimization of Protein Intake in Pre-Dialysis Chronic Kidney Disease Patients, Nutrients, 2022, vol. 14, no. 6, article no. 1182, DOI: 10.3390/nu14061182 particularly, abstract, second paragraph in section of 5. conclusions | 1-10 |
| Y | KITAKAZE, T. et al., Lactoferrin promotes murine C2C12 myoblast proliferation and differentiation and myotube hypertrophy, Molecular Medicine Reports, 2018, vol. 17, pages 5912-5920, DOI: 10.3892/mmr.2018.8603 abstract | 1-10 |
| X | 岩本千奈, 他3名, ラクトフェリン投与による慢性腎臓病およびその合併症の進行抑制効果, 日本腎臓学会誌, 24 May 2022, vol. 64, no. 3, page 221, O-001, (Jpn. J. Nephrol.), non-official translation (IWAMOTO, China and 3 others, Effect of lactoferrin administration on suppressing the progression of chronic kidney disease and its complications) 特に、方法の項、結果の項、考察の項, non-official translation (particularly, sections of methods, results, discussion) | 1-7, 9, 10 |
| A | | 8 |

**INTERNATIONAL SEARCH REPORT**

| International application No. |
|---|
| **PCT/JP2024/017376** |

**Box No. I     Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

   a. ☑ forming part of the international application as filed.

   b. ☐ furnished subsequent to the international filing date for the purposes of international search (Rule 13*ter*.1(a)),

   ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

## INTERNATIONAL SEARCH REPORT
### Information on patent family members

International application No.

**PCT/JP2024/017376**

| Patent document cited in search report | Publication date (day/month/year) | Patent family member(s) | Publication date (day/month/year) |
|---|---|---|---|
| JP 2014-227375 A | 08 December 2014 | (Family: none) | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YUNG-HO HSU et al.** Lactoferrin Contributes a Renoprotective Effect in Acute Kidney Injury and Early Renal Fibrosis. *Pharmaceutics*, 2020, vol. 12, 434

- **NOEL J. M. CANO et al.** Application of Branched-Chain Amino Acids in Human Pathological States: Renal Failure. *J Nutr.*, January 2006, vol. 136 (1), 299S-307S